# EUROPÄISCHE PATENTANMELDUNG

(11) **EP 1 072 260 A1**
(43) Veröffentlichungstag der Anmeldung: **31.01.2001**
(21) Anmeldenummer: 00114978.0
(22) Anmeldetag: 20.07.2000
(51) Int. Cl.: A61K 9/70, A61P 13/10, A61K 47/44

(54) **Transdermalsysteme zur Abgabe von Muscarin-Rezeptor-Antagonisten und ihre Verwendung zur Behandlung von Spasmen der glatten Muskulatur im urologischen Bereich**

(30) Priorität: 22.07.1999 DE 19934523
(71) Anmelder: Novosis Pharma AG, 80807 München (DE)
(72) Erfinder: Fischer, Wilfried, Dr., 80807 München (DE); Piecha, Thomas, Dr., 80807 München (DE)
(74) Vertreter: Biagosch, Stephan (DE)

(57) **Zusammenfassung**

Die Erfindung betrifft Transdermalsysteme zur Abgabe von Muscarin-Rezeptor-Antagonisten, die dadurch gekennzeichnet sind, daß sie eine Zusammensetzung aus einem Muscarin-Rezeptor-Antagonisten als Wirkstoff, Teebaumöl und einem mit Wasser mischbaren Lösungsmittel enthalten. Die Erfindung gibt ferner die Verwendung derartiger Transdermalsysteme zur Behandlung von Spasmen der glatten Muskulatur im urologischen Bereich an.

## Beschreibung

Die Erfindung betrifft Transdermalsysteme zur Abgabe von Muscarin-Rezeptor-Antagonisten und ihre Verwendung zur Behandlung von Spasmen der glatten Muskulatur im urologischen Bereich.

### Einleitung und Stand der Technik

Heutzutage werden Spasmen der glatten Muskulatur im Bereich des Magen-Darm-Kanals, des Harnsystems und der weiblichen Geschlechtsorgane mit spezifischen Antispasmolytika behandelt. Trospiumchlorid, Atropin, Scopolamin, N-Butylscopolaminiumbromid, Tropicamid, Oxyphenoniumbromid und Methantheliniumbromid sind Beispiele dafür. Sie zählen zur Gruppe der Muscarin-Rezeptor-Antagonisten und hemmen kompetitiv die peripheren Wirkungen von Acetylcholin, die normalerweise durch Erregung muscarinischer Acetylcholin-Rezeptoren zustande kommen. Sie seien beispielhaft für ein spezifisches Antispasmolytikum genannt.

Im Plasma treten ungefähr 5 Stunden nach oraler Gabe und 42 min nach einer intravenösen Gabe von Trospiumchlorid die Spitzenkonzentrationen auf. Die mittlere Eliminationshalbwertzeit liegt im Bereich von 5 bis 21 Stunden.

Trospiumchlorid wirkt zuverlässig bei der Behandlung von Pollakisurie und Nykturie, bei nicht hormonell und nicht organisch bedingten vegetativen Blasenfunktionsstörungen (Reizblase, sensorische Dranginkontinenz) und bei Harnblasenkrämpfen infolge supranukleärer Läsion des Rückenmarks. Außerdem wird es zur Erleichterung endoskopischer Untersuchungen und zur Funktionsdiagnostik bei Untersuchungen des Gastrointestinaltraktes eingesetzt.

Als Nebenwirkungen können Magen-Darm-Störungen Miktionsstörungen, Störungen der Herzfrequenz sowie Hemmungen der Speichel- und Schweißsekretion auftreten. Am Auge wird durch Trospiumchlorid die parasympathische Stimulation des Musculus sphincter pupillae und des Musculus ciliaris aufgehoben, so daß es zu einer Mydriasis und einer Lähmung der Akkommodationsfähigkeit (Zykloplegie) kommen kann.

Die Dosierung von Trospiumchlorid liegt oral bei 30 bis 45 mg dreimal täglich im Abstand von 8 Stunden und parenteral bei 1 bis 2 mg (langsame Injektion i. v.) einmal täglich. Sie richtet sich nach dem Schweregrad der Erkrankung und dem Therapieefolg im Laufe der medikamentösen Behandlung. Orale Einzeldosen bis 120 mg werden bei gesunden Probanden ohne meßbare Veränderung von Blutdruck, EKG, Pupillendurchmesser und Speichelfluß vertragen

Die chemische Bezeichnung für Trospiumchlorid ist endo-3-[(Hydroxydiphenylacetyl)oxy]spiro[8-azoniabicyclo[3.2.1]octan-8,1'-pyrrolidin]chlorid.

Die Summenformel ist C₂₅H₃₀ClNO₃, was eine Molekülmasse von 427,97 ergibt.

Die Resorption nach oraler Gabe ist gering und niedriger als die von Atropin.

Trospiumchlorid wird, bei einer renalen Clearance von 807 ml/min., größtenteils unverändert ausgeschieden, davon ein geringer Teil als Spiroalkohol nach Abspaltung des Benzilsäurerestes. Metabolisierung (oral): 1,6% von 5 mg Trospiumchlorid werden innerhalb von 48 h eliminiert, 4,39% von 45 mg Trospiumchlorid werden innerhalb von 48 h eliminiert. Metabolisierung (i. v.): 55,37% von 1,2 mg Trospiumchlorid werden innerhalb von 48 h eliminiert, 49,6% von 2 mg Trospiumchlorid werden innerhalb von 48 h eliminiert.

Die Wirkungsdauer von Trospiumchlorid ist kürzer als die von Atropin, die absolute Bioverfügbarkeit liegt zwischen 3 und 6%. Die Gesamtclearance beträgt 1405 ml/min.

Im Hinblick auf eine Langzeittherapie bei Blasenfunktionsstörungen und cyclisch mehrtägig auftretende Spasmen bei Dysmenorrhoe gilt es, eine für die Patienten angenehmere und zuverlässige Applikationsart zur Verfügung zu stellen.

Die transdermale Verabreichung von hochwirksamen Substanzen, wie Hormonen, starken Analgetika, Nitraten etc., ist seit einigen Jahren bekannt. Transdermalsysteme zur Abgabe von Muscarin-Rezeptor-Antagonisten sind bisher aber nicht bekannt.

Trospiumchlorid wird unter dem Handelsnamen Spasmex® in zur Injektion geeigneter Form, in Form von Tabletten zur oralen Verabreichung oder in Form von Suppositorien zur rektalen Verabreichung vertrieben. Eine zur transdermalen Applikation geeignete Form existiert derzeit nicht.

In der EP 856312 wird die Verwendung von Trospiumchlorid zur Herstellung einer Arzneizubereitung auf Basis einer sterilen, wäßrigen Lösung zur Behandlung von Blasenfunktionsstörungen und des Harnsystems beschrieben, wobei die Applikation über einen Blasenkatheter intravesikal direkt in die Blase hinein erfolgt und die wäßrige Lösung mit einem pH-Wert ≥ 4,5 vor der Applikation zubereitet wird

In der DE 19612504 Al 970821 (9739) der Fa. Boehringer Ingelheim KG, Deutschland, wird eine Zubereitung zur Behandlung von Morbus Alzheimer beschrieben, die im Vergleich zu herkömmlichen Zubereitungen geringere Nebenwirkungen zeigt: "A composition comprises a muscarine agonist and a peripherally active anti-muscarine. Preferably the muscarine agonist is talsaclidine and the peripherally active anti-muscarine is pirenzepine, ipratropium bromide, oxitropium bromide, N-butylscopolaminium bromide, trospiumchloride, methantheline bromide, thiotropium bromide or BEA 2108. USE: The composition is used to treat Alzheimer's disease (claimed). ADVANTAGE: The peripherally active anti-muscarine reduces excess salivation caused by use of talsaclidine alone"

Im Patent DE 4041559 A 920625 (9227) der Fa. Boehringer Ingelheim KG, Deutschland, wird die Herstellung von Zubereitungen beschrieben, welche die Nebenwirkungen auf das zentrale und periphere Nervensystem bei der Verabreichung von Opiaten verringern: "Anticholinergic agents containing quaternary ammonium compounds are used for production of medicament preparations for limitation of side effects of opiates/opioids. Analgesic compounds containing the anticholinergic agents and an opiate/opioid are also new. The anticholinergic agent is especially butyl(nor)scopolaminium bromide, N-methyl-scopolaminium bromide, trospiumchloride or cimetropium chloride, and the opiate/opioid is morphine, biprenorphine, hydrocodone or heroin. USE/ADVANTAGE: The anticholinergic agents are used to prevent side effects on the peripheral and central nervous systems and in the gastrointestinal tract leading to colics. Dosage is esp. 15-25 (esp. 20) mg., i. m. or i. v."

In der DE 3604575 A 860828 (8636) der Fa. Degussa AG, Deutschland, wird eine Zubereitung für die Behandlung von Spasmen im urologischen und gastrointestinalen Bereich beschrieben: "New compositions contain flupirtin, i.e 2-amino-3-ethoxycarbonyl amino-6-(4-fluorobenzylamino)-pyridine (I), and at least one anticholinergic spasmolytic (II) with an analgesic activity component. (I) and (II) may be present as acid-addition salts. (II) is a butylscopolamine, fenpiverinium, trospium, pramiverin or ciclonium salt. The (I) : (II) wt. ratio is 1:0,05-150, esp. 1:0.1-100. The compositions, in unit dosage form, contain 10-900 (esp. 10-600) mg (I) and 0.3-100 mg (II), esp. 30-400 mg (I) and 3-60 mg butylscopolamine or 10-900 mg (I) and 5-60 ciclonium base. USE/ADVANTAGE: The compositions are useful as analgesics and spasmolytic agents for treating spastic pain, biliary colic, biliary spasm, biliary dyskinesia, renal colic, pain in the urinary tract, spastic pain in the gastrointestinal tract, tenesmus, dysenorrhoea, etc. The compositions have synergistically increased analgesic and spasmolytic activity."

In R. Brandau/B. H. Lippold, Dermal and Transdermal Absorption, Wissenschaftliche Verlagsgesellschaft mbH Stuttgart, 1982, S. 42 wird die erhöhte Permeationsfähigkeit von Substanzen mit amphiphilen Eigenschaften durch das Stratum corneum beschrieben. Trospiumchlorid enthält ein quartäres Ammoniumion und ist sehr hydrophil. Erfahrungsgemäß permeieren Verbindungen, die ein quartäres Ammoniumion enthalten, sehr schlecht durch die Haut.

Aufgabe der nachstehend beschriebenen Erfindung ist, ein zuverlässig wirkendes und hautverträgliches Transdermalsystem zur Abgabe von Muscarin-Rezeptor-Antagonisten zur Verfügung zu stellen.

Diese Aufgabe wird erfindungsgemäß mit einem Transdermalsystem zur Abgabe von Muscarin-Rezeptor-Antagonisten gelöst, das dadurch gekennzeichnet ist, daß es eine Zusammensetzung aus einem Muscarin-Rezeptor-Antagonisten als Wirkstoff, Teebaumöl und einem mit Wasser mischbaren Lösungsmittel enthält.

Eine bevorzugte Ausgestaltung des erfindungsgemäßen Transdermalsystems ist dadurch gekennzeichnet, daß es sich bei dem Muscarin-Rezeptor-Antagonisten um eine oder mehrere der Verbindungen Trospiumchlorid, Atropin, Scopolamin, N-Butylscopolaminiumbromid, Tropicamid, Oxyphenoniumbromid oder Methantheliniumbromid handelt.

Eine weitere bevorzugte Ausgestaltung des erfindungsgemäßen Transdermalsystems ist dadurch gekennzeichnet, daß es sich bei dem mit Wasser mischbaren Lösungsmittel um Ethanol handelt.

Eine weitere bevorzugte Ausgestaltung des erfindungsgemäßen Transdermalsystems ist dadurch gekennzeichnet, daß es sich um eine Zusammensetzung aus 15 bis 85 Teilen Teebaumöl und 85 bis 15 Teilen Ethanol (Teebaumöl und Ethanol ergeben zusammen insgesamt 100 Teile), besonders bevorzugt um eine Zusammensetzung aus 15 Teilen Teebaumöl und 85 Teilen Ethanol, handelt, in der eine oder mehrere der Verbindungen Trospiumchlorid, Atropin, Scopolamin, N-Butylscopolaminiumbromid, Tropicamid, Oxyphenoniumbromid oder Methantheliniumbromid bis zur Sättigung gelöst ist/sind.

Eine weitere bevorzugte Ausgestaltung des erfindungsgemäßen Transdermalsystems ist dadurch gekennzeichnet, daß die Zusammensetzung außerdem einen zur Herstellung von Gelen, Salben oder Cremes geeigneten pharmazeutisch akzeptablen Träger enthält.

Eine weitere bevorzugte Ausgestaltung des erfindungsgemäßen Transdermalsystems ist dadurch gekennzeichnet, daß die Zusammensetzung aus einem Muscarin-Antagonisten als Wirkstoff, Teebaumöl und einem mit Wasser mischbaren Lösungsmittel in einer Schicht eines flächigen selbstklebenden Pflasters mit mehrschichtigem Aufbau enthalten ist.

Eine weitere bevorzugte Ausgestaltung des erfindungsgemäßen Transdermalsystems ist dadurch gekennzeichnet, daß neben der die Wirkstoffzusammensetzung enthaltenden Schicht eine Abdeckung, eine Klebstoffschicht und auf der der Abdeckung gegenüberliegenden Seite ein die Klebstoffschicht temporär abdeckender und abziehbarer Träger vorgesehen sind.

Eine weitere bevorzugte Ausgestaltung des erfindungsgemäßen Transdermalsystems ist dadurch gekennzeichnet, daß die Wirkstoffzusammensetzung in der Klebstoffschicht enthalten ist.

Eine weitere bevorzugte Ausgestaltung des erfindungsgemäßen Transdermalsystems ist dadurch gekennzeichnet, daß die Abdeckung aus Kunststoffolie, Gewebe oder Vlies besteht.

Eine weitere bevorzugte Ausgestaltung des erfindungsgemäßen Transdermalsystems ist dadurch gekennzeichnet, daß der Träger aus Kunststoffolie, Papier oder einem Laminat daraus besteht.

Eine weitere bevorzugte Ausgestaltung des erfindungsgemäßen Transdermalsystems ist dadurch gekennzeichnet, daß die Zusammensetzung aus einem Muscarin-Rezeptor-Antagonisten als Wirkstoff, Teebaumöl und einem mit Wasser mischbaren Lösungsmittel in einem von einer mikroporösen, wirkstoffdurchlässigen Membran gebildeten Reservoir eines flächigen selbstklebenden Pflasters enthalten ist.

Eine weitere bevorzugte Ausgestaltung des erfindungsgemäßen Transdermalsystems ist dadurch gekennzeichnet, daß die wirkstoffdurchlässige Membran aus Celluloseacetat, Polyester oder Polypropylen besteht.

Eine weitere bevorzugte Ausgestaltung des erfindungsgemäßen Transdermalsystems ist dadurch gekennzeichnet, daß die wirkstoffdurchlässige Membran eine Porengröße von 0,1 bis 0,2 µm hat.

Eine weitere bevorzugte Ausgestaltung des erfindungsgemäßen Transdermalsystems ist dadurch gekennzeichnet, daß neben dem die Wirkstoffzusammensetzung enthaltenden Reservoir eine wirkstoffundurchlässige Abdeckung, ein das Reservoir umgebender Klebstoffring und ein auf der der Abdeckung gegenüberliegenden Seite des Reservoirs liegender, temporär abdeckender und entfernbarer Träger vorgesehen sind.

Eine weitere bevorzugte Ausgestaltung des erfindungsgemäßen Transdermalsystems ist dadurch gekennzeichnet, daß die Abdeckung aus Kunststoffolie besteht.

Eine weitere bevorzugte Ausgestaltung des erfindungsgemäßen Transdermalsystems ist dadurch gekennzeichnet, daß der Träger aus silikonisierter Kunststoffolie oder silikonisiertem Papier besteht.

Eine weitere bevorzugte Ausgestaltung des erfindungsgemäßen Transdermalsystems ist dadurch gekennzeichnet, daß es sich bei der Kunststoffolie um Polyester-, Polyethylen- oder Polypropylenfolie handelt.

Die Erfindung gibt ferner die Verwendung des oben definierten Transdermalsystems zur Behandlung von Spasmen der glatten Muskulatur im urologischen Bereich an.

### Detaillierte Beschreibung der Erfindung am Beispiel Trospiumchlorid.

Das erfindungsgemäße Transdermalsystem gestattet eine bessere Permeation von Trospiumchlorid durch die Haut durch die Verwendung einer Kombination aus Teebaumöl und einem mit Wasser mischbaren Lösungsmittel.

Es wurde überraschenderweise in Versuchen zur Verbesserung der Resorption gefunden, daß mit dieser Kombination ein Vielfaches der Menge an Trospiumchlorid durch die Haut permeiert als üblicherweise bei gut permeierenden Substanzen zu erwarten wäre. Ferner war die Tatsache überraschend, daß Trospiumchlorid aus dem üblicherweise als Resorptionsförderer (z. B. bei Sexualhormonen oder Analgetika wie Fentanyl) verwendeten Alkohol ebenfalls weitaus besser permeiert als zu erwarten gewesen wäre.

Nachstehend wird die Erfindung durch Abbildungen näher erläutert. Es zeigen
- Abb. 1: eine Trospiumchlorid-Permeation aus Ethanol, Teebaumöl und deren Kombination über 9 h und
- Abb. 2: eine weitere Trospiumchlorid-Permeation aus Ethanol, Teebaumöl und deren Kombination über 21 h.

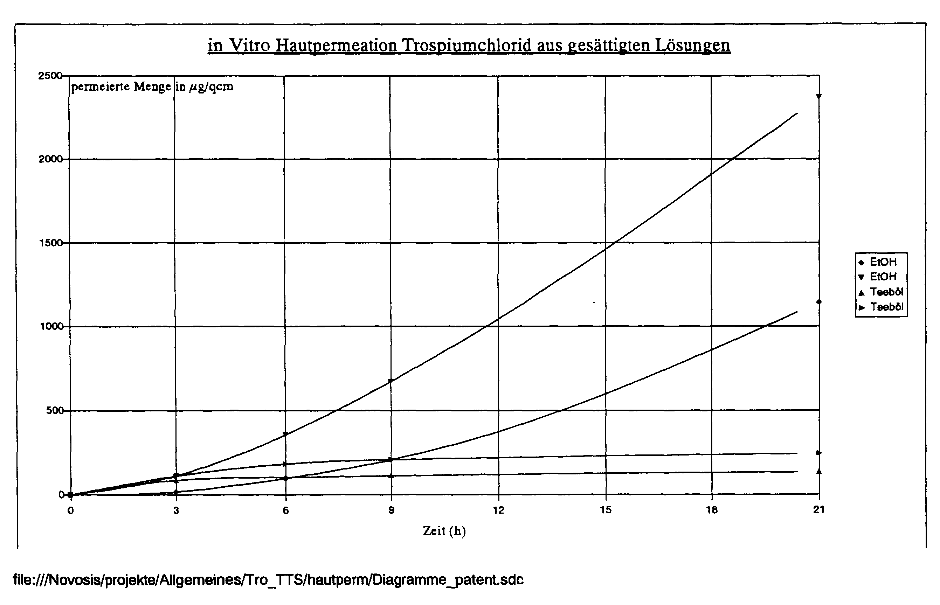

In der Abbildung 1 ist das Ausmaß der Permeation von Trospiumchlorid durch die Haut in vitro unter Verwendung von jeweils gesättigten Lösungen von Trospiumchlorid in Ethanol, Teebaumöl und in der Kombination Ethanol/Teebaumöl dargestellt. Es wurden gesättigte Lösungen verwendet, um jeweils maximale thermodynamische Aktivitäten zu erzielen.

Abbildung 1 zeigt, daß sich die beste Permeation durch die Haut bei Verwendung des Trospiumchlorids in Ethanol, Teebaumöl und in einem Gemisch aus EtOH und Teebaumöl ergibt.

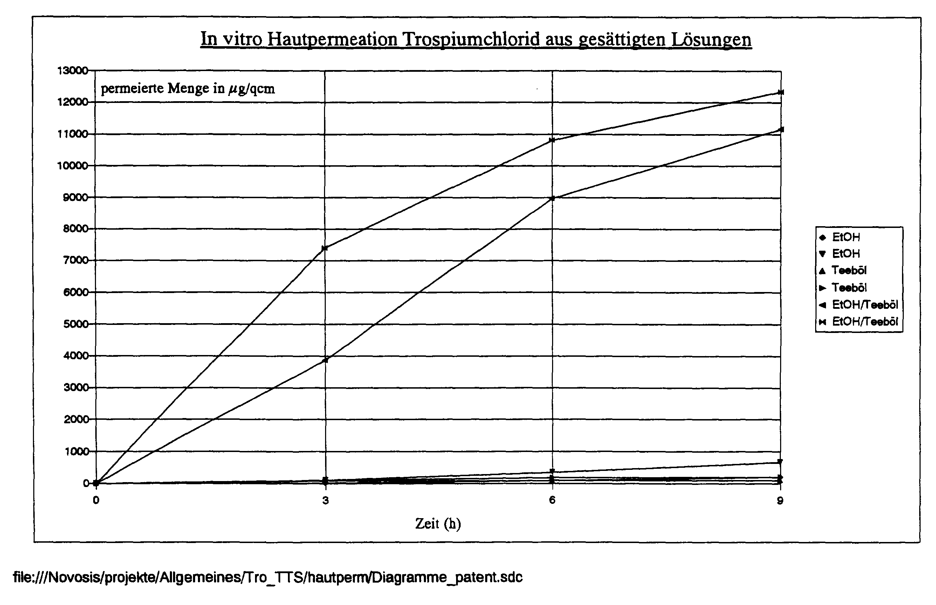

Die Abbildung 2 zeigt, daß sich die permeierte Menge bei der Verwendung von Teebaumöl nach 9 Stunden einem Sättigungswert nähert. Die permeierte Menge bei Verwendung von ethanolischer Lösung übersteigt diesen Wert bei weitem. Dagegen verändert sich das Permeationsverhalten bei der Verwendung einer Kombination aus Ethanol und Teebaumöl unerwartet. Für die permeierte Menge wäre ein Wert zu erwarten gewesen, der sich zwischen den Werten bei Verwendung von Ethanol oder Teebaumöl einstellt. Überraschenderweise wird jedoch der unter Verwendung einer ethanolischen Lösung erhaltene Wert im Mittel um den Faktor 13 übertroffen.

Die erfindungsgemäßen Lösungen mit Trospiumchlorid als Wirkstoff können in gel- salben- oder cremeartige Trägersysteme (Transdermalsysteme im weiteren Sinn) eingearbeitet und in flächigen Transdermalsystemen im engeren Sinn, d. h. sog. "transdermalen therapeutischen Systemen (TTS)", z. B. Wirkstoffpflastern, oder in Reservoirsystemen (Reservoir-TTS) verwendet werden.

Bevorzugte Ausführungsformen sind Transdermalsysteme im engeren Sinn, insbesondere solche mit Reservoir. Diese Transdermalsysteme bestehen aus einer wirkstoffundurchlässigen Abdeckung bzw. Deckfolie (Backing-Foil), die aus Polyester, Polypropylen, Polyethylen o.ä. besteht. Die Backing-Foil bildet zusammen mit einer mikroporösen Membran, die den Wirkstoffdurchtritt kontrollieren kann oder keine Diffsionskontrolle ausübt, ein beutelförmiges Reservoir, in das die erfindungsgemäßen Wirkstofflösungen eingefüllt werden. Nach dem Einfüllen wird die Backing-Foil mit der Membran verschweißt oder verklebt und anschließend gegebenenfalls eine Klebstoffschicht aufgebracht, die das Transdermalsystem auf der Haut fixiert. Statt der Klebstoffschicht kann auch ein Klebstoffring angebracht werden, so daß die Membran direkt auf der Haut aufliegt.

Im folgenden wird die Erfindung anhand von Beispielen erläutert.

### Beispiel 1

### Wirkstofflösung

In einem Gemisch aus 15 Teilen Teebaumöl und 85 Teilen Ethanol wird Trospiumchlorid bis zur Sättigung gelöst.

### Reservoir-TTS

Mit einem geeigneten thermischen Schweißgerät wird eine heißsiegelfähig beschichtete Polyesterfolie mit einer Stärke von 19 µm und einer kreisförmigen Fläche von 30 cm² mit einer Celluloseacetatmembran mit einer Porenweite von ca. 0,1 µm am Rand so verschweißt, daß eine Einfüllöffnung verbleibt, durch die 1 ml der o. g. Wirkstofflösung in den entstandenen Beutel eingefüllt wird. Anschließend wird die Einfüllöffnung ebenfalls verschweißt. Es entsteht eine kreisförmige Fläche von 20 cm², durch die membranseitig die Wirkstofflösung herausdiffundieren kann. Zur Fixierung auf der Haut wird über den Rand des Flüssigkeitsreservoirs ein Klebstoffring montiert, der aus einem druckempfindlichen Haftklebstoff, z. B. Duro-Tak 386-2287 (Fa. National, NL-Zutphen), auf einer 19 µm starken Polyesterfolie, z. B. Hostaphan MN 19 (Mitsubishi Foils, D-Frankfurt), besteht. Das gesamte System wird auf eine silikonisierte Polyesterfolie oder silikonisiertes Papier (Release-Liner) montiert. Der Release-Liner wird vor der Applikation auf die Haut entfernt und verworfen.

## Patentansprüche

1. Transdermalsystem zur Abgabe von Muscarin-Rezeptor-Antagonisten, dadurch gekennzeichnet, daß es eine Zusammensetzung aus einem Muscarin-Rezeptor-Antagonisten als Wirkstoff, Teebaumöl und einem mit Wasser mischbaren Lösungsmittel enthält.

2. Transdermalsystem nach Anspruch 1, dadurch gekennzeichnet, daß es sich bei dem Muscarin-Rezeptor-Antagonisten um eine oder mehrere der Verbindungen Trospiumchlorid, Atropin, Scopolamin, N-Butylscopolaminiumbromid, Tropicamid, Oxyphenoniumbromid oder Methantheliniumbromid handelt.

3. Transdermalsystem nach einem der vorherigen Ansprüche, dadurch gekennzeichnet, daß es sich bei dem mit Wasser mischbaren Lösungsmittel um Ethanol handelt.

4. Transdermalsystem nach einem der Ansprüche 1 bis 3, dadurch, gekennzeichnet, daß es sich um eine Zusammensetzung aus 15 bis 85 Teilen Teebaumöl und 85 bis 15 Teilen Ethanol (Teebaumöl und Ethanol ergeben zusammen insgesamt 100 Teile), besonders bevorzugt um eine Zusammensetzung aus 15 Teilen Teebaumöl und 85 Teilen Ethanol, handelt, in der eine oder mehrere der Verbindungen Trospiumchlorid, Atropin, Scopolamin, N-Butylscopolaminiumbromid, Tropicamid, Oxyphenoniumbromid oder Methantheliniumbromid bis zur Sättigung gelöst ist/sind.

5. Transdermalsystem nach einem der vorherigen Ansprüche, dadurch gekennzeichnet, daß die Zusammensetzung außerdem einen zur Herstellung von Gelen, Salben oder Cremes geeigneten pharmazeutisch akzeptablen Träger enthält.

6. Transdermalsystem nach einem der Ansprüche 1 bis 4, dadurch gekennzeichnet, daß die Zusammensetzung aus einem Muscarin-Rezeptor-Antagonisten als Wirkstoff, Teebaumöl und einem mit Wasser mischbaren Lösungsmittel in einer Schicht eines flächigen selbstklebenden Pflasters mit mehrschichtigem Aufbau enthalten ist.

7. Transdermalsystem nach Anspruch 6, dadurch gekennzeichnet, daß neben der die Wirkstoffzusammensetzung enthaltenden Schicht eine Abdeckung, eine Klebstoffschicht und auf der der Abdeckung gegenüberliegenden Seite ein die Klebstoffschicht temporär abdeckender und abziehbarer Träger vorgesehen sind.

8. Transdermalsystem nach Anspruch 7, dadurch gekennzeichnet, daß die Wirkstoffzusammensetzung in der Klebstoffschicht enthalten ist.

9. Transdermalsystem nach Anspruch 7 oder 8, dadurch gekennzeichnet, daß die Abdeckung aus Kunststoffolie, Gewebe oder Vlies besteht.

10. Transdermalsystem nach einem der Ansprüche 7 bis 9, dadurch gekennzeichnet, daß der Träger aus Kunststoffolie, Papier oder einem Laminat daraus besteht.

11. Transdermalsystem nach einem der Ansprüche 1 bis 4, dadurch gekennzeichnet, daß die Zusammensetzung aus einem Muscarin-Rezeptor-Antagonisten als Wirkstoff, Teebaumöl und einem mit Wasser mischbaren Lösungsmittel in einem von einer mikroporösen, wirkstoffdurchlässigen Membran gebildeten Reservoir eines flächigen selbstklebenden Pflasters enthalten ist.

12. Transdermalsystem nach Anspruch 11, dadurch gekennzeichnet, daß die wirkstoffdurchlässige Membran aus Celluloseacetat, Polyester oder Polypropylen besteht.

13. Transdermalsystem nach Anspruch 11 oder 12, dadurch gekennzeichnet, daß die wirkstoffdurchlässige Membran eine Porengröße von 0,1 bis 0,2 µm hat.

14. Transdermalsystem nach einem der Ansprüche 11 bis 13, dadurch gekennzeichnet, daß neben dem die Wirkstoffzusammensetzung enthaltenden Reservoir eine wirkstoffundurchlässige Abdeckung, ein das Reservoir umgebender Klebstoffring und ein auf der der Abdeckung gegenüberliegenden Seite des Reservoirs liegender, temporär abdeckender und entfernbarer Träger vorgesehen sind.

15. Transdermalsystem nach Anspruch 14, dadurch gekennzeichnet, daß die Abdeckung aus Kunststoffolie besteht.

16. Transdermalsystem nach Anspruch 14 oder 15, dadurch gekennzeichnet, daß der Träger aus silikonisierter Kunststoffolie oder silikonisiertem Papier besteht.

17. Transdermalsystem nach Anspruch 15 oder 16, dadurch gekennzeichnet, daß es sich bei der Kunststoffolie um Polyester-, Polyethylen- oder Polypropylenfolie handelt.

18. Verwendung eines Transdermalsystems nach einem der vorheriggen Ansprüche zur Behandlung von Spasmen der glatten Muskulatur im urologischen Bereich.
